# EUROPEAN PATENT APPLICATION

(11) **EP 0 923 944 A1**
(43) Date of publication of application: **23.06.1999**
(21) Application number: 97908553.7
(22) Date of filing: 31.03.1997
(51) Int. Cl.: A61K 49/00, C12N 9/88, C12N 15/60

(54) **DIAGNOSTIC COMPOSITION FOR DISEASES ACCOMPANIED BY AUTOIMMUNE REACTIONS CAUSED BY 65K-GLUTAMIC ACID DECARBOXYLASE**

(30) Priority: 01.04.1996 JP 7887896
(71) Applicant: CHUGAI SEIYAKU KABUSHIKI KAISHA, Tokyo, 115-8543 (JP)
(72) Inventor: KURE, Shigeo, Sendai-shi, Miyagi 981-0917 (JP); NARISAWA, Kuniaki, Sendai-shi, Miyagi 981-0911 (JP); MUTO, Yoshiko, Sendai-shi, Miyagi 982-0821 (JP); SAKATA, Yoshiyuki, Sendai-shi, Miyagi 981-0933 (JP); SATOH, Jo, Sendai-shi, Miyagi 981-0942 (JP)
(74) Representative: Harding, Charles Thomas
(86) International application number: JP9701104
(87) International publication number: WO9736618

(57) **Abstract**

This invention provides a composition for diagnosis through skin reaction of a disease involving an autoimmune reaction induced with 65K-glutamic acid decarboxylase (GAD65), which comprises GAD65 or a partial peptide thereof that can react with T cells in the skin and/or on the surface of the skin of a mammal to produce dermal inflammation in the mammal, and provides the use of said enzyme or partial peptide thereof for such diagnosis, and further, it provides a method of diagnosing a disease involving an autoimmune reaction induced with GAD65 by the steps of applying said enzyme or a partial peptide thereof in the skin and/or on the surface of the skin of a subject to cause skin reaction between GAD65 or the partial peptide thereof and T cells in the subject and determining the size of the dermal inflammation produced by the skin reaction, as well as a method of estimating cell-mediated immunity against GAD65 comprising the stated steps. According to this invention, the diagnosis of a disease involving an autoimmune reaction induced with 65K-glutamic acid decarboxylase can be accomplished through simple operations by performing estimation of the cell-mediated immunity against GAD65.

## Description

### TECHNICAL FIELD

This invention relates to a method of diagnosing a disease involving an autoimmune reaction induced with 65K-glutamic acid decarboxylase and to a composition for the diagnosis.

### BACKGROUND ART

In routine medical examinations and treatments, insulin-dependent diabetes mellitus is diagnosed on the basis of test results such as on blood glucose, urine glucose and those from glucose tolerance tests. Since these tests are mostly concerned with tests reflecting the pathologic conditions of insulin deficiency, it is difficult to diagnose the disease by the use of these tests at its early stage when the secretion of insulin is not quite impaired. At present, it is recognized that insulin-dependent diabetes mellitus is an endocrine disease in which pancreatic β cells are destroyed by the autoimmune mechanism to cause the absolute deficiency of insulin. Thus, it has been expected that the introduction of tests for detecting this autoimmune process would enable the diagnosis at earlier stages. Recently, one of the autoantigens was identified to be glutamic acid decarboxylase abundantly existing in the brain, and some attempts were initiated to detect the immune reactions against this molecule. (Nature 347: 151-156, 1990.) Glutamic acid decarboxylase is an enzyme that generates a neurotransmitter, γ-aminobutyric acid from glutamic acid. This enzyme occurs in two types of isozymes, one having a molecular weight of 65 kDa and the other having a molecular weight of 67 kDa; they are referred to as "65K-glutamic acid decarboxylase (GAD65)" and "67K-glutamic acid decarboxylase (GAD67)," respectively. Later studies have revealed that GAD65 induces stronger autoimmune reactions. (J. Clin. Invest. 1993; 91: 2084-2090.) Among the immune reactions against GAD65, humoral immune reactions were comparatively easy to detect, so screening was initially directed to anti-GAD65 antibodies. A large number of patients suffering from insulin-dependent diabetes mellitus were tested for the antibody titers in their sera, and 69% of the patients who were within one year after the onset of the disease was found to be positive for the anti-GAD65 antibodies. (Diabetes 41: 548-551, 1992.) Further, a high percentage of the first degree kin of the patients who had not developed diabetes mellitus were antibody positive, a fact leading to the possibility of diagnosis before the onset of the disease: The kin were considered a high-risk group in respect of the pathopoiesis of insulin-dependent diabetes mellitus. (Proc. Natl. Acad. Sci. USA 89: 9841-9845, 1992.)

Methods to measure the anti-GAD65 antibody titers employ ELISA (enzyme linked immunosorbent assay), RIA (radioimmuno assay), immunoprecipitation, and immunoblotting, etc. These methods, among others, utilize the following as antigens: (1) GAD65 purified from porcine brain; (2) GAD65 that is expressed in insect cells with the aid of a baculovirus vector and then purified; and (3) GAD65 that is expressed in *E. coli* and then purified. Some of these GAD65's have already been commercialized. Depending on differences in the types of antigens or in the methods of measurement, sensitivity and normal cutoff values vary.

There have been reported many cases that indicate that the anti-GAD65 antibody titers are useful for the diagnosis of insulin-dependent diabetes mellitus. On the other hand, their limitations have been pointed out as the result of screening the sera in a large number of diabetes mellitus patients. One limitation is that cases of high antibody titers do not necessarily require insulin therapy within a short period of time. This means that fluctuations in the antibody titer can not be used to differentiate between good prognosis and poor prognosis. Rather, it has been reported that many of the low antibody titer cases do require insulin at early stages. (Lancet 341: 1565-1569, 1993.) The cause for this finding has been presumed to be ascribable to the fact that pancreatic β cells are in fact destroyed by cell-mediated immunity rather than humoral immunity.

In insulin-dependent diabetes mellitus, there is a pathological type in which non-insulin-dependent diabetes mellitus initially develops at an early stage of the disease and then undergoes transition to insulin-dependent diabetes mellitus as the insulin secretion is gradually impaired: slow-progressing type insulin-dependent diabetes mellitus. This type of diabetes mellitus is numerously found in this country and presumed to account for about five percent of the non-insulin-dependent diabetes mellitus cases. (Diab TES CARE, 1993; 16: 780-788 and Internal Medicine (Naika) 1995; 76: 31-35.) It has been suggested that in these cases the measurement of the anit-GAD65 antibody titers at an early stage of the disease is useful for accurate diagnosis of the disease; however, even under these circumstances, cases of high antibody titers do not necessarily result in insulin deficiency at earlier stages of the disease. This suggests that in such cases a full understanding of the kinetics of not only humoral immunity but also cell-mediated immunity is important in the prognosis of the disease as well as in the choice of a suitable treatment protocol.

However, the screening for cell-mediated immunity is seldom performed in routine medical examinations and treatments, because the detection of cell-mediated immunity requires more complicated techniques than the detection of humoral immunity. The most representative method is one that utilizes T cells' nature to initiate their division when subjected to specific antigen stimulation; this phenomenon is referred to as T cell blastogenesis (or T cell proliferation). When T cells are divided, they incorporate thymidine that serves as a material for DNA synthesis. The amount of the incorporated thymidine can be calculated if tritium-labeled radioactive thymidine is mixed with the medium. When this method is used to diagnose human, mononuclear cells are first separated from about 10 ml of peripheral blood by density-gradient centrifugation. Next, the cells are grown for three to seven days in a suitable medium containing a partial peptide of GAD65, and then they are further grown for approximately 18 h after addition of radioactive thymidine. Subsequently, the amount of the incorporated thymidine is calculated by collecting cell fractions and measuring the radioactivity in the fractions; the amount can be used as an index of cell-mediated immunity.

This currently used method for determining cell-mediated immunity based on the incorporation of radioactive thymidine finds the following difficulties if it is to be applied to the actual diagnosis:
1. About one hour is needed to separate mononuclear cells from human peripheral blood and this step also requires aseptic manipulations. Therefore, testing facilities and techniques are indispensable that will allow the aseptic manipulations and subsequent culturing and these cannot be done in ordinary clinics and outpatient hospitals of small sizes. In addition, because viable cells must be separated, blood samples cannot be stored for a prolonged period and the transportation of the blood samples also involves difficulties.
2. Use of radioactive thymidine is only permitted within specified controlled areas. Thus its use is not practically feasible in ordinary clinical laboratories. Although methods have been devised that utilize fluorescent labeled thymidine instead of radioactive substances, they are inferior to the radioactive thymidine method in sensitivity and, thus, have not gained a very wide use.
3. The required quantity of blood is as much as 10 ml even at the minimum, and this poses a problem in blood collection for the testing of infants who assume a major proportion of patients with insulin-dependent diabetes mellitus.
4. A medium for the mononuclear cells derived from the peripheral blood of patients normally contains a fetal bovine serum. The reactivity of the mononuclear cells with the fetal bovine serum scatters between individual fetal bovine sera and thus, the results of testing conducted in different facilities cannot simply be compared. This has proved an obstacle to standardization of the testing.

Accordingly, this invention aims at providing a composition with which the diagnosis of a disease involving an autoimmune reaction induced with 65K-glutamic acid decarboxylase can be accomplished by performing the estimation of cell-mediated immunity against GAD65 through simple operations.

Further, an object of this invention is to provide a method of estimating cell-mediated immunity against GAD65 through simple operations as well as to provide a method that utilizes said method to diagnoses a disease involving an autoimmune reaction induced with 65K-glutamic acid decarboxylase.

### DISCLOSURE OF INVENTION

In order to achieve the above-mentioned objects, the present inventors injected a partial peptide of GAD65 in the skin of NOD mice which served as model animals for spontaneous development of insulin-dependent diabetes mellitus. Two days after the injection, the skin at the injection sites was examined and changes such as swelling and redness were noted. This suggests that it is possible to estimate cell-mediated immunity based on the skin reaction caused by administration of the partial peptide of GAD65. The present invention has been accomplished based on this finding.

Specifically, this invention provides a composition for diagnosis through skin reaction of a disease involving an autoimmune reaction induced with 65K-glutamic acid decarboxylase (GAD65), which comprises GAD65 or a partial peptide thereof that can react with T cells in the skin and/or on the surface of the skin of a mammal to produce dermal inflammation in the mammal.

Also, the invention provides a method of diagnosing a disease involving an autoimmune reaction induced with GAD65 by the steps of applying GAD65 or a partial peptide thereof in the skin and/or on the surface of the skin of a subject to cause skin reaction between GAD65 or the partial peptide thereof and T cells in the subject and determining the size of the dermal inflammation produced by the skin reaction, said GAD65 or partial peptide thereof being capable of reacting with T cells in the skin and/or on the surface of the skin of a mammal to produce dermal inflammation in the mammal.

Further, the invention provides a method of estimating cell-mediated immunity against GAD65 by the steps of applying GAD65 or a partial peptide thereof in the skin and/or on the surface of the skin of a subject to cause skin reaction between GAD65 or the partial peptide thereof and T cells in the subject and determining the size of the dermal inflammation produced by the skin reaction, said GAD65 or partial peptide thereof being capable of reacting with T cells in the skin and/or on the surface of the skin of a mammal to produce dermal inflammation in the mammal.

The invention also provides the use of GAD65 or a partial peptide thereof for diagnosis through skin reaction of a disease involving an autoimmune reaction induced with GAD65, said GAD65 or partial peptide thereof being capable of reacting with T cells in the skin and/or on the surface of the skin of a mammal to produce dermal inflammation in the mammal.

In addition, the invention provides the use of GAD65 or a partial peptide thereof for preparation of a composition for diagnosis through skin reaction of a disease involving an autoimmune reaction induced with GAD65, said GAD65 or partial peptide thereof being capable of reacting with T cells in the skin and/or on the surface of the skin of a mammal to produce dermal inflammation in the mammal.

### BRIEF DESCRIPTION OF DRAWINGS

FIG. 1 is a graph showing the time dependence of footpad swelling in NOD mice administered with a partial peptide of GAD65.
FIG. 2 is a graph showing the footpad swelling in NOD mice induced by administration of various peptide antigens (partial peptide of GAD65, cyclophilin, and carboxyl carrier protein).
FIG. 3 is a graph showing the footpad swelling in different strains of mouse (NOD, C3H/He, C57BL, Balb/c, B1O.A, and DBA) induced by the partial peptide of GAD65.
FIG. 4 is a graph showing T cell blastogenesis in different strains of mouse (NOD, C3H/He, C57BL, Balb/c, B1O.A, and DBA) by administration of the partial peptide of GAD65.
FIG. 5 is a graph showing the correlation between the footpad swelling reaction and the T cell blastogenesis in different strains of mouse (NOD, C3H/He, C57BL, Balb/c, B1O.A, and DBA) by administration of the partial peptide of GAD65.

### BEST MODE FOR CARRYING OUT THE INVENTION

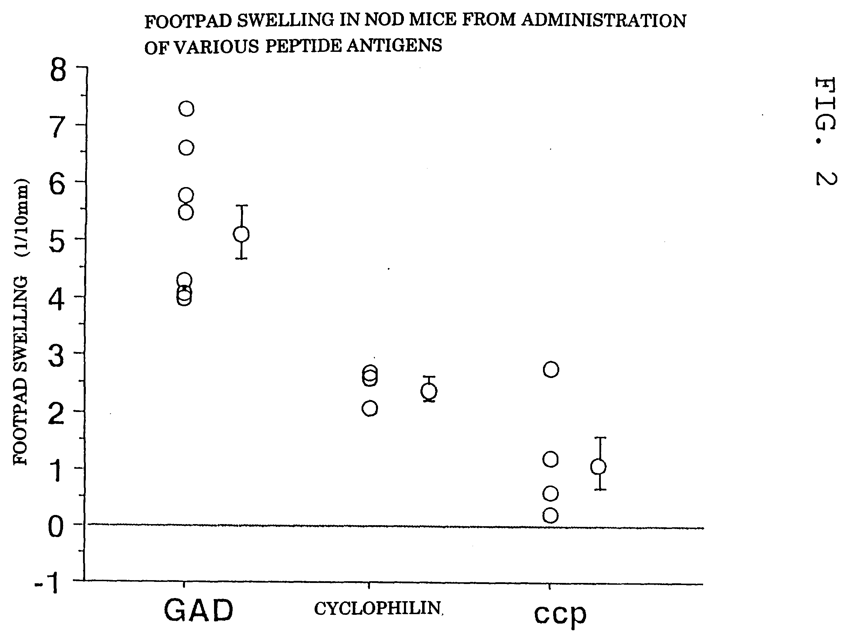

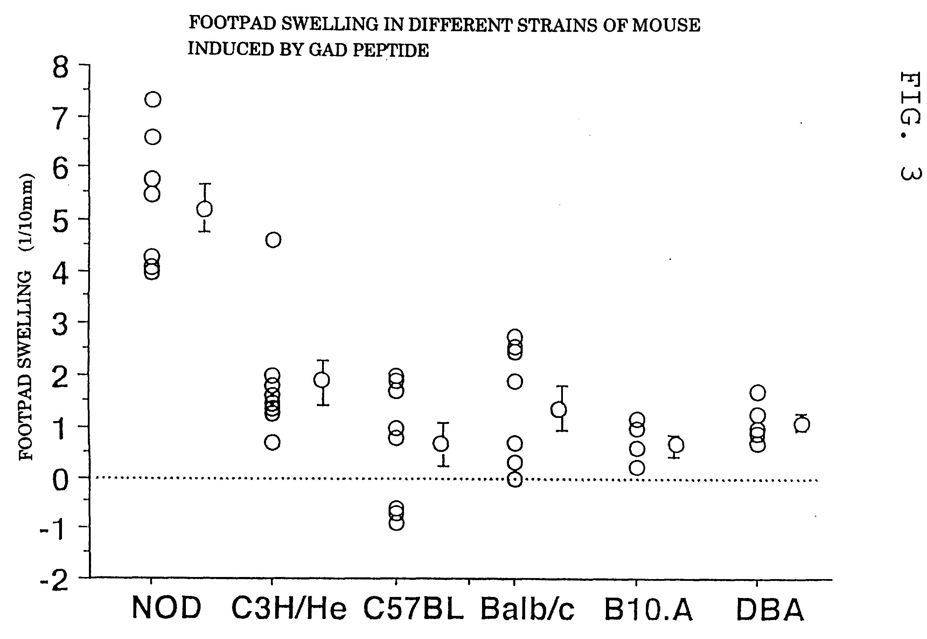

GAP (glutamic acid decarboxylase) is an enzyme that functions in the synthesis of an inhibitory neurotransmitter γ-aminobutyric acid from glutamic acid, and it mainly occurs in high concentration at the γ-aminobutyric acid activatable neuronal nerve ending in the brain. It is a known substance also existing in pancreatic island cells, thyroid glands, adrenal cortex, testis, oviduct, and the wall of the stomach, etc. GAD occurs in two types of isoforms, GAD67 and GAD65, and in this invention, GAD65 comprising 585 amino acid residues with a molecular weight of 65,000 is to be used. Human, rat, murine, and other GAD65's have been isolated and their respective amino acid sequences have already been elucidated. (See, for example, FIG. 2 in Proc. Natl. Acad. USA 89, 2115-2119, 1992; FIG. 3 in Neuron, 7, 91-100, 1991; and FIG. 2 in Biochemica Biophysica Acta, 1216, 157-160, 1993.) Since the amino acid sequences for GAD65 are highly homologous among human, rat, and murine GAD65's, any of these can be used in this invention. From the standpoint of early diagnosis and prognosis of human insulin-dependent diabetes mellitus, human GAD65 which is an inherent recognition antigen of human T cells is preferably employed to improve the detection sensitivity. In this invention, GAD65 may not necessarily be a protein having the full amino acid sequence. In other words, it may be a partial peptide such as one selected from within the amino acid sequence ranging up to about 120 amino acids in GAD65 as counted from the C-terminus. Two examples are a peptide comprising the amino acid sequence of SEQ ID NO: 1 and a peptide comprising the amino acid sequence of SEQ ID NO: 2. In consideration of the peptide length that is easy to synthesize chemically by the solid phase method, this invention preferably employs a mixture of peptides of 10-30 amino acid residues, more preferably peptides of 20-26, which are selected within the amino acid sequence ranging up to about the 120 amino acids in GAD65 as counted from the C-terminus. Examples are peptides comprising the amino acid sequences of SEQ ID NO: 3 through NO: 6. These peptides correspond respectively to the amino acid sequence from positions 473 to 498 in the partial peptide of human GAD65 set forth in SEQ ID NO: 2, the amino acid sequence from positions 494 to 518 in the same partial peptide, the amino acid sequence from positions 514 to 538, and the amino acid sequence from positions 534 to 558. The amino acid sequence from positions 508 to 509 in the amino acid sequence of the partial peptide of human GAD65 set forth in SEQ ID NO: 2 is Tyr-Ile, while the murine amino acid sequence for the corresponding site (the sequence from positions 9 to 10 in the amino acid sequence set forth in SEQ ID NO: 1) is Phe-Val. Thus, the amino acid sequence may accordingly be changed to this latter sequence.

These GAD65's and their peptides can be obtained by isolation and purification from biological materials such as porcire brain, rat brain and human brain as described in Biochem. J. 231, 695-703, 1985; Eur. J. Biochem. 86, 143-152, 1978; J. Neurochem. 56, 720-723, 1991; J. Biochem. 248, 3029-3034, 1973; Proc. Natl. Acad. Sci. USA, 83, 8808-8812, 1986; and Proc. Natl. Acad. Sci. USA, 89, 9841-9845, 1992. Alternatively, they can be easily obtained by genetic engineering techniques as described in Eur. J. Biochem. 212, 597-603, 1993; Proc. Natl. Acad. Sci. USA, 90, 2832-2836, 1993; and J. Exp. Med. 180, 1979-1984, 1994. In this case, in order to facilitate the isolation and purification of the expressed GAD65 or its partial peptide, a peptide comprising six to seven consecutive histidine residues may preliminarily be bound to the N-terminus as effected in an Example that follows.

For a mixture of peptides comprising about 30 amino acid residues, each peptide can easily be obtained by chemical synthesis using the solid phase method.

GAD65 or its partial peptide may adopt any forms of preparations insofar as they can be administered in the skin and/or on the surface of the skin of a mammal such as human; however, they are preferably in dosage forms that permit subcutaneous administration by injection or the like. Specific examples of such forms include those which have a diagnostically effective amount, typically 25-75 weight %, preferably 40-60 weight % of GAD65, dissolved or suspended in a physiological saline solution or the like. Furthermore, potassium alum can be added to the preparations in order to prevent GAD65 from diffusing in the skin. For this purpose, three components -- 10% potassium alum solution, 10% sodium carbonate solution, and the antigen peptide -- are mixed to form a precipitate, which in turn is resuspended in a physiological saline solution for subsequent use. In this case, the amount of potassium alum to be finally contained in the injectable preparation is 0.2-0.4%.

In this invention, from the viewpoint of ease for GAD65 to remain localized in the skin and/or on the surface of the skin, the solubility of GAD65 or its partial peptides in the preparations is preferably low and, therefore suspensions of GAD65 or its partial peptide are preferred.

In this invention, the above-mentioned preparations are preferably used after their pH is adjusted to the range of from 6.0 to 7.0.

A composition comprising GAD65 or its partial peptide is applied to the skin and/or on the surface of the skin of an individual subject, and the size of the dermal inflammation produced by the skin reaction between GAD65 or its partial peptide and T cells is determined; this will enable the early diagnosis and prognosis of diseases involving the autoimmune reaction induced with GAD65 such as insulin-dependent diabetes mellitus, Stiffman syndrome, and certain kinds of autoimmune thyropathy. Examples of the individual subjects to be examined include mammals such as a human, mouse, and rat.

For example, the diagnosis of insulin-dependent diabetes mellitus (IDDM) can be performed according to the following protocol. Namely, the GAD65 or its partial peptide as described above is applied in the skin of an individual subject by subcutaneous injection or the like. Thereafter, typically after two days, any change on the skin such as swelling or redness is determined. Alternatively, the skin may be scratched and the GAD65 or its partial peptide adhered to the skin (scratch test). Thereafter typically after 48 hours, any change on the skin such as swelling or redness is determined. Such change on the skin as swelling or redness is believed to be caused by the following mechanism. After GAD65 or its partial peptide has been incorporated into antigen presenting cells and subjected to processing, the antigen stimulation is conveyed to T cells. As a result, the T cells are activated to produce inflammations, which involve the release of a variety of cytokines and the mobilization of groups of mononuclear and multinucleate cells.

As will be shown in the Examples given below, the degree of such change on the skin as swelling or redness has good correspondence with the IDDM diagnosis based on the currently practised incorporation of radioactive thymidine. Therefore, if the correlation between the degree of the change such as the size of redness or the degree of induration and the symptoms of IDDM is determined in advance, convenient diagnosis will be possible.

This invention will be further explained in detail by means of examples hereinbelow; however, the scope of the invention is not to be limited to these examples.

In order to illustrate the utility of skin reaction utilizing partial peptides of GAD65, experiments were conducted using NOD mice serving as model animals that for spontaneous development of insulin-dependent diabetes mellitus (Exp. Anim. 29, 1-13, 1980.). In the case of human, the peptide is first injected subcutaneously to the flexor surface of a forearm similarly to the tuberculin test and estimation may then be performed by examining the degree of redness or induration on the skin. Otherwise, the evaluation may be performed by a scratch test. In the experiments with mice to be described below, skin reaction was examined using footpads where the subcutaneous connective tissue was dense enough to prevent smooth diffusion of the injected preparation.

### EXAMPLE 1

### Preparation of Partial Peptide of Murine GAD65

A partial peptide containing 86 amino acid residues at the C-terminus of murine GAD65 (Biochem. Biophysica Acta. 1216, 157-160, 1993) was prepared as described below.
Method for Preparation of Murine GAD65 Peptide. PolyA⁺ RNA was isolated from mouse brain, and cDNA was synthesized using an oligo dT primer and reverse transcriptase. (For this procedure, the method described in J. Clin. Invest. 90, 160-164, 1992 was followed.) With a solution of this cDNA used as a template, PCR was performed using the following two kinds of chemically synthesized primers:
Positive-oriented primer: 5'-
   CAGACATATGGCTCAACACACAAATGTCTGCTTC-3' (SEQ ID NO: 7)
Reverse-oriented primer: 5'-
   CGGAATTCGGATCCTGAAACAGTTTGATGAGTGAGG-3' (SEQ ID NO: 8)

The resulting PCR product was doubly digested with restriction enzymes BamHI and NdeI and a DNA fragment (about 300 bp) was isolated by agarose gel electrophoresis. Subsequently, the DNA was purified using a DNA purification kit (available from Qiagen Inc.).

An expression vector pET14b (available from Novagen Inc., U.S.A.) was doubly digested with restriction enzymes BamHI and NdeI and ligated with the previously obtained DNA fragment of ca. 300 bp using a DNA ligase. A vector into which the thus cyclized DNA fragment had been incorporated was transferred into *E. coli* BL21 and the strain was subjected to expression. Specifically, this *E. coli* strain was grown in an ampicillin-supplemented L-broth medium at 37 °C until the absorbance at 600 nm reached 0.7 and thereafter IPTG was added to give a final concentration of 0.5 mM. The culturing was further continued at 37 °C for 3 h, and then cells were harvested.

Purification of the desired protein from these cells was conducted using a His-Bind Resin and His-Bind Buffer Kit, both of which were available from Novagen Inc. According to the attached protocols, particularly the procedure for the case where a protein is present in the inclusion body, the purification was conducted on a nickel column in the presence of 6M urea utilizing six consecutive histidine residues existing at the N-terminus of the expressed protein. The resulting protein proved to comprise 109 amino acid residues such that a peptide derived from the expression vector pET14b which comprised 23 amino acid residues as described below was bound to the N-terminus of the partial peptide derived from murine GAD65 as set forth in SEQ ID NO: 1 (86 amino acid residues).

Met Gly Ser Ser His His His His His His Ser Ser Gly Leu Val Pro Arg Gly Ser His Met Val
(MGSSHHHHHHSSGLVPRGSHMV-) (SEQ ID NO: 9)

The peptide solution eluted from the column was dialyzed against phosphate buffered saline (PBS). Because the peptide was insolubilized during the dialysis, precipitates were collected by centrifugation and resuspended in PBS. The peptide can then be stored as such at -80 °C for a prolonged period.

Since the peptide turned insoluble in water during the purification step, this suspension was used in the following experiments.

### EXAMPLE 2

### Time-dependence of Footpad Swelling in NOD Mice

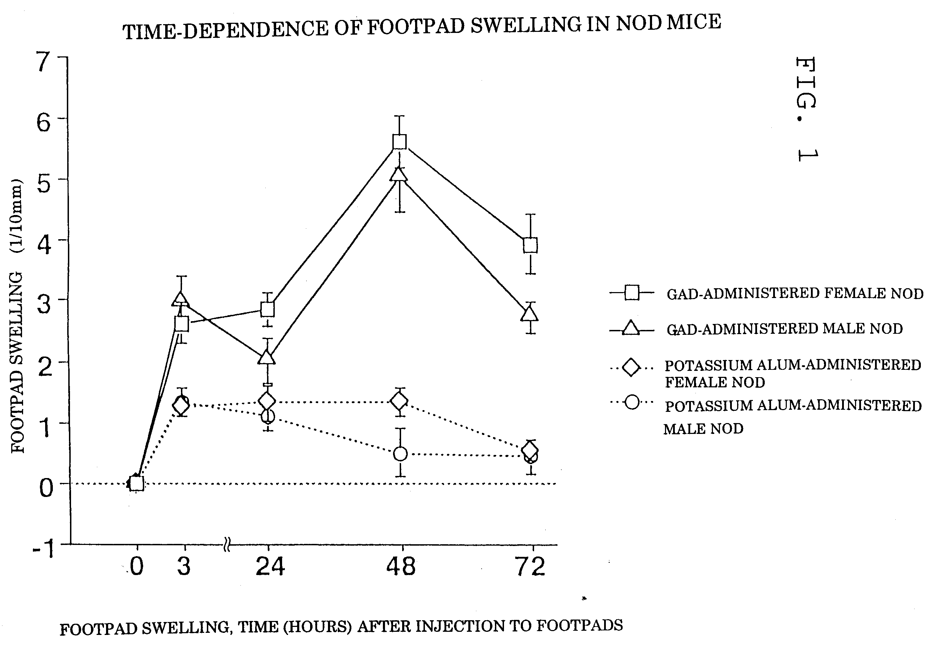

After 10 µg of the partial peptide of GAD65 was mixed with 10% potassium alum solution, the pH was adjusted to 6.0-6.3 with 10% sodium bicarbonate solution. The resulting precipitates were washed with a physiological saline solution and finally, resuspended in 20 µl of a physiological saline solution. The resuspension was subcuteniously injected to the footpads in the left hind paws of NOD mice. The swelling that would be observed after the injection was evaluated by measuring the thickness of the hind paws with precision vernier calipers. The results are shown in FIG. 1 (i.e., the solid lines in FIG. 1). As a control, only the precipitates formed from potassium alum with sodium carbonate were injected to the right hind paws of the same individual animals. The swelling that would be observed after the injection was evaluated (i.e., the dotted lines in FIG. 1). Swelling with peaks at 48 h of the injections was observed in the hind paws of the mice that received the injections of the partial peptide of GAD65. No sex difference was recognized in the swelling reaction with the partial peptide of GAD65.

### EXAMPLE 3

### Footpad Swelling upon Administration of Various Peptide Antigens

In order to determine whether the swelling in the hind paws observed in Example 2 was a reaction specific to the partial peptide of GAD65, two kinds of peptides other than the partial peptide of GAD65 were used in the experiments. Rat cyclophilin (MW: 12,000) and *E. coli* carboxyl carrier protein (CCP; MW: 22,000) were selected, both of which had no homology to GAD65 in their primary structures. In a like manner to the case of expression of the partial peptide of GAD65 (Example 1), the expression vector pET14b was used to express peptides in *E. coli* in such a form that six histidine residues were present at the N-terminius, and then their purification was conducted using nickel chelate columns. The two purified peptides were injected to the hind paws of NOD mice, and 48 h after the injection, the swelling in the hind paws was measured. As shown in FIG. 2, it was ascertained that the reaction against the partial peptide of GAD65 was significantly stronger than that against cyclophilin or CCP.

### EXAMPLE 4

### Footpad Swelling Induced by the Partial Peptide of GAD65 in Different Strains of Mouse

An investigation was made to see whether or not a skin reaction against the partial peptide of GAD65 would occur in mouse strains that do not spontaneously develop diabetes mellitus -- C3H/He, C57BL, Balb/c, B1O.A -- and a mouse strain that spontaneously develops non-insulin-dependent diabetes metillus, DBA. According to a method similar to that of Example 2, four to eight animals (12-16 weeks of age) of different strains were subjected to footpad tests with the partial peptide of GAD65. The results are shown in FIG. 3, the NOD mice that spontaneously develop diabetes mellitus displayed significantly stronger reactions than mice of the other strains.

### EXAMPLE 5

### T Cell Blastogenesis during the Antigen Stimulation by the Partial Peptide of GAD65

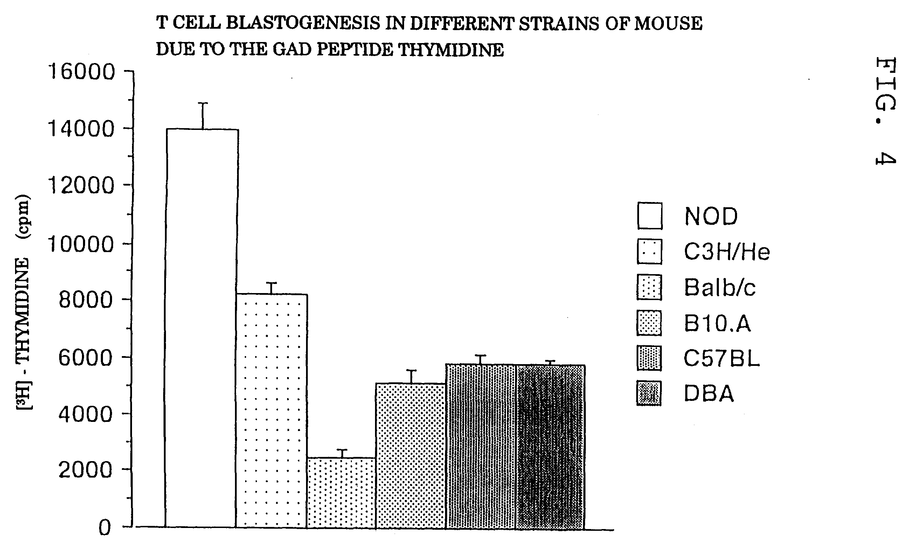

A partial peptide of GAD65 similar to that used in Example 2 was employed as a stimulating antigen and the incorporation of radioactive thymidine was measured. (J. Clin. Invest. 94, 2125-2129, 1994; J. Exp. Med. 180, 1979-1984, 1994.) The mice used were not only a NOD mouse but also four strains that do not spontaneously develop insulin-dependent diabetes mellitus -- C3H/He, Balb/c, B1O.A, and C57BL -- and a strain that spontaneously develops non-insulin-dependent diabetes mellitus, DBA, totalling to six kinds of mouse. As FIG.4 shows, the NOD mouse that spontaneously develops insulin-dependent diabetes metillus could incorporate a significantly higher amount of radioactive thymidine than mice of any other strain. This clearly indicated that the cell-mediated immunologcal reaction in the NOD mouse against the partial peptide of GAD65 was stronger than that in mice of any other strain.

### EXAMPLE 6

### Correlation between the Footpad Swelling Reaction and the Incorporation Reaction of Radioactive Thymidine

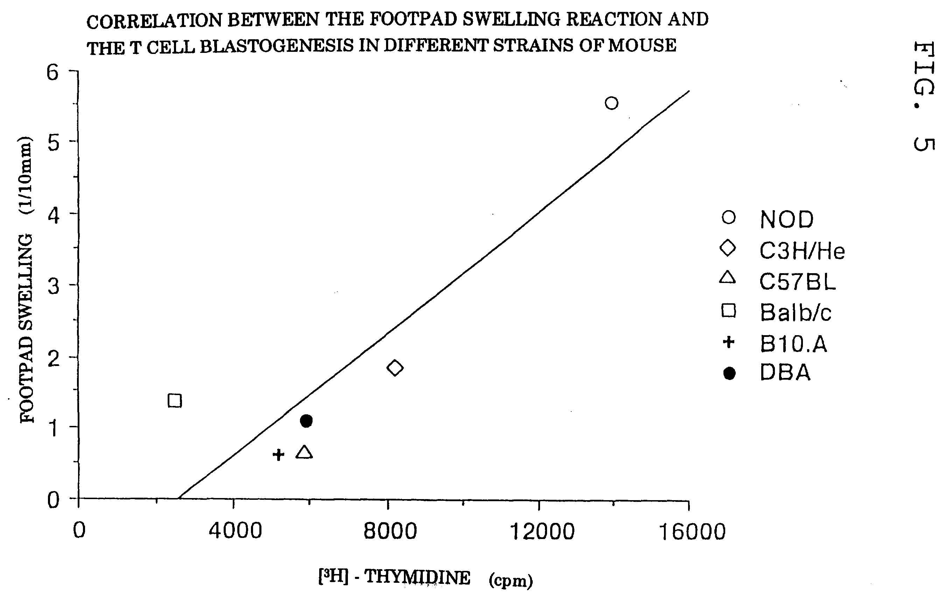

The correlation between the amount of incorporation of radioactive thymidine (the experiment in Example 5) and the results of the intracutaneous reaction (the experiment in Example 4) was investigated: the thymidine incorporation represents an existing method for estimating cell-mediated immunity and the intracutaneous reaction represents a method of estimate cell-mediated immunity according to this invention. As FIG. 5 shows, a significant correlationship was recognized between the two experiments, where the correlation coefficient (R) was 0.809 and p value was 0.05 or less. Accordingly, it was found that the intracutaneous reaction method based on GAD65 could replace the conventional method based on the incorporation of radioactive thymidine as a technique for reliable estimation of cell-mediated immunoreaction.

### INDUSTRIAL APPLICABILITY

The composition comprising GAD65 or a partial peptide thereof according to this invention is applied in the skin and/or on the surface of the skin of an individual subject, and any change on the skin such as swelling or redness (delayed hypersensitivity reaction) is examined after the lapse of an appropriate period; these very simple operations enable estimation of the cell-mediated immunity against the mentioned antigen. This enables both pre-onset diagnosis and post-onset prognosis of insulin-dependent diabetes mellitus and other diseases that have so far defied the practice of these approaches by the sole measurement of humoral immunity. Furthermore, the method of the invention requires less trouble than the conventional measurement of humoral immunity and, hence, it has the potential to be used extensively for the purpose of screening school children or patients suffering from non-insulin-dependent diabetes mellitus.

## Claims

1. A composition for diagnosis through skin reaction of a disease involving an autoimmune reaction induced with 65K-glutamic acid decarboxylase (GAD65), which comprises GAD65 or a partial peptide thereof that can react with T cells in the skin and/or on the surface of the skin of a mammal to produce dermal inflammation in the mammal.

2. The composition of claim 1, wherein the disease involving an autoimmune reaction induced with GAD65 is insulin dependent diabetes mellitus.

3. The composition of claim 1, wherein the mammal is human.

4. The composition of claim 1, wherein GAD65 is derived from human, a rat or a mouse.

5. The composition of claim 1, wherein the partial peptide of GAD65 is a peptide selected from within the amino acid sequence ranging from the C-terminus to the 120th amino acid of the enzyme.

6. The composition of claim 1, wherein the partial peptide of GAD65 is a mixture of peptides of 10-30 amino acid residues within the amino acid sequence ranging from the C-terminus to the 120th amino acid of the enzyme.

7. The composition of claim 1, wherein the partial peptide of GAD65 comprises the amino acid sequence of SEQ ID NO:1.

8. The composition of claim 1, wherein the partial peptide of GAD65 comprises the amino acid sequence of SEQ ID NO:2.

9. The composition of claim 1, which is in a form of suspension.

10. The composition of claim 9, wherein the suspension is a physiological saline suspension.

11. The composition of claim 9, which further comprises potassium alum.

12. The composition of claim 1, which is used in a diagnosis of a disease involving an autoimmune reaction induced with GAD65 by the steps of applying the composition in the skin and/or on the surface of the skin of a subject to cause skin reation between GAD65 or a partial peptide thereof and T cells in the subject and determining the size of the dermal inflammation produced by the skin reaction.

13. A method of diagnosing a disease involving an autoimmune reaction induced with GAD65 by the steps of applying GAD65 or a partial peptide thereof in the skin and/or on the surface of the skin of a subject to cause skin reation between GAD65 or the partial peptide thereof and T cells in the subject and determining the size of the dermal inflammation produced by the skin reaction, said GAD65 or partial peptide thereof being capable of reacting with T cells in the skin and/or on the surface of the skin of a mammal to produce dermal inflammation in the mammal.

14. A method of estimating cell-mediated immunity against GAD65 by the steps of applying GAD65 or a partial peptide thereof in the skin and/or on the surface of the skin of a subject to cause skin reation between GAD65 or the partial peptide thereof and T cells in the subject and determining the size of the dermal inflammation produced by the skin reaction, said GAD65 or partial peptide thereof being capable of reacting with T cells in the skin and/or on the surface of the skin of a mammal to produce dermal inflammation in the mammal.

15. Use of GAD65 or a partial peptide thereof for diagnosis through skin reaction of a disease involving an autoimmune reaction induced with GAD65, said GAD65 or partial peptide thereof being capable of reacting with T cells in the skin and/or on the surface of the skin of a mammal to produce dermal inflammation in the mammal.

16. Use of GAD65 or a partial peptide thereof for preparation of a composition for diagnosis through skin reaction of a disease involving an autoimmune reaction induced with GAD65, said GAD65 or partial peptide thereof being capable of reacting with T cells in the skin and/or on the surface of the skin of a mammal to produce dermal inflammation in the mammal.


SEQUENCE LISTING

<110> Chugai Seiyaku Kabushiki Kaisha

<120> Composition for Diagnosis of a disease involving an autoimmune
      reaction induced with 65K-Glutamic Acid Decarboxylase

<130> PH-377PCT-EP

<150> JP 078878/1996
<151> 1996-04-01

<160> 9

<210> 1
<211> 86
<212> PRT
<213> Mus musculus

<400> 1
Gln His Thr Asn Val Cys Phe Trp Phe Val Pro Pro Ser Leu Arg
 1               5                   10                  15
Thr Leu Glu Asp Asn Glu Glu Arg Met Ser Arg Leu Ser Lys Val
                20                  25                  30
Ala Pro Val Ile Lys Ala Arg Met Met Glu Tyr Gly Thr Thr Met
                35                  40                  45
Val Ser Tyr Gln Pro Leu Gly Asp Lys Val Asn Phe Phe Arg Met
                50                  55                  60
Val Ile Ser Asn Pro Ala Ala Thr His Gln Asp Ile Asp Phe Leu

65                  70                  75

Ile Glu Glu Ile Glu Arg Leu Gly Gln Asp Leu

                80                  85

<210> 2

<211> 585

<212> PRT

<213> Homo sapiens

<400> 2

Met Ala Ser Pro Gly Ser Gly Phe Trp Ser Phe Gly Ser Glu Asp

                5                   10                  15

Gly Ser Gly Asp Ser Glu Asn Pro Gly Thr Ala Arg Ala Trp Cys

                20                  25                  30

Gln Val Ala Gln Lys Phe Thr Gly Gly Ile Gly Asn Lys Leu Cys

                35                  40                  45

Ala Leu Leu Tyr Gly Asp Ala Glu Lys Pro Ala Glu Ser Gly Gly

                50                  55                  60

Ser Gln Pro Pro Arg Ala Ala Ala Arg Lys Ala Ala Cys Ala Cys

                65                  70                  75

Asp Gln Lys Pro Cys Ser Cys Ser Lys Val Asp Val Asn Tyr Ala

                80                  85                  90

Phe Leu His Ala Thr Asp Leu Leu Pro Ala Cys Asp Gly Glu Arg

                95                  100                 105

Pro Thr Leu Ala Phe Leu Gln Asp Val Met Asn Ile Leu Leu Gln

                110                 115                 120

Tyr Val Val Lys Ser Phe Asp Arg Ser Thr Lys Val Ile Asp Phe

                125                 130                 135

His Tyr Pro Asn Glu Leu Leu Gln Glu Tyr Asn Trp Glu Leu Ala

                140                 145                 150
Asp Gln Pro Gln Asn Leu Glu Glu Ile Leu Met His Cys Gln Thr
                155                 160                 165
Thr Leu Lys Tyr Ala Ile Lys Thr Gly His Pro Arg Tyr Phe Asn
                170                 175                 180
Gln Leu Ser Thr Gly Leu Asp Met Val Gly Leu Ala Ala Asp Trp
                185                 190                 195
Leu Thr Ser Thr Ala Asn Thr Asn Met Phe Thr Tyr Glu Ile Ala
                200                 205                 210
Pro Val Phe Val Leu Leu Glu Tyr Val Thr Leu Lys Lys Met Arg
                215                 220                 225
Glu Ile Ile Gly Trp Pro Gly Gly Ser Gly Asp Gly Ile Phe Ser
                230                 235                 240
Pro Gly Gly Ala Ile Ser Asn Met Tyr Ala Met Met Ile Ala Arg
                245                 250                 255
Phe Lys Met Phe Pro Glu Val Lys Glu Lys Gly Met Ala Ala Leu
                260                 265                 270
Pro Arg Leu Ile Ala Phe Thr Ser Glu His Ser His Phe Ser Leu
                275                 280                 285
Lys Lys Gly Ala Ala Ala Leu Gly Ile Gly Thr Asp Ser Val Ile
                290                 295                 300
Leu Ile Lys Cys Asp Glu Arg Gly Lys Met Ile Pro Ser Asp Leu
                305                 310                 315
Glu Arg Arg Ile Leu Glu Ala Lys Gln Lys Gly Phe Val Pro Phe
                320                 325                 330
Leu Val Ser Ala Thr Ala Gly Thr Thr Val Tyr Gly Ala Phe Asp
                335                 340                 345
Pro Leu Leu Ala Val Ala Asp Ile Cys Lys Lys Tyr Lys Ile Trp
                350                 355                 360

Met His Val Asp Ala Ala Trp Gly Gly Gly Leu Leu Met Ser Arg
                365                 370                 375
Lys His Lys Trp Lys Leu Ser Gly Val Glu Arg Ala Asn Ser Val
                380                 385                 390
Thr Trp Asn Pro His Lys Met Met Gly Val Pro Leu Gln Cys Ser
                395                 400                 405
Ala Leu Leu Val Arg Glu Glu Gly Leu Met Gln Asn Cys Asn Gln
                410                 415                 420
Met His Ala Ser Tyr Leu Phe Gln Gln Asp Lys His Tyr Asp Leu
                425                 430                 435
Ser Tyr Asp Thr Gly Asp Lys Ala Leu Gln Cys Gly Arg His Val
                440                 445                 450
Asp Val Phe Lys Leu Trp Leu Met Trp Arg Ala Lys Gly Thr Thr
                455                 460                 465
Gly Phe Glu Ala His Val Asp Lys Cys Leu Glu Leu Ala Glu Tyr
                470                 475                 480
Leu Tyr Asn Ile Ile Lys Asn Arg Glu Gly Tyr Glu Met Val Phe
                485                 490                 495
Asp Gly Lys Pro Gln His Thr Asn Val Cys Phe Trp Tyr Ile Pro
                500                 505                 510
Pro Ser Leu Arg Thr Leu Glu Asp Asn Glu Glu Arg Met Ser Arg
                515                 520                 525
Leu Ser Lys Val Ala Pro Val Ile Lys Ala Arg Met Met Glu Tyr
                530                 535                 540
Gly Thr Thr Met Val Ser Tyr Gln Pro Leu Gly Asp Lys Val Asn
                545                 550                 555
Phe Phe Arg Met Val Ile Ser Asn Pro Ala Ala Thr His Gln Asp
                560                 565                 570
Ile Asp Phe Leu Ile Glu Glu Ile Glu Arg Leu Gly Gln Asp Leu

                575                 580                 585

<210> 3
<211> 26
<212> PRT
<213> Homo sapiens

<400>

Lys Cys Leu Glu Leu Ala Glu Tyr Leu Tyr Asn Ile Ile Lys Asn
                5                   10                  15
Arg Glu Gly Tyr Glu Met Val Phe Asp Gly Lys
20

<210> 4
<211> 25
<212> PRT
<213> Homo sapiens

<400> 4

Val Phe Asp Gly Lys Pro Gln His Thr Asn Val Cys Phe Trp Phe
1               5                   10                  15
Val Pro Pro Ser Leu Arg Thr Leu Glu Asp
                20                  25

<210> 5
<211> 25
<212> PRT
<213> Homo sapiens

<400> 5

Arg Thr Leu Glu Asp Asn Glu Glu Arg Met Ser Arg Leu Ser Lys

1               5                   10                  15

Val Ala Pro Val Ile Lys Ala Arg Met Met

                20                  25

<210> 6

<211> 25

<212> PRT

<213> Homo sapiens

<400> 6

Lys Ala Arg Met Met Glu Tyr Gly Thr Thr Met Val Ser Tyr Gln

1               5                   10                  15

Pro Leu Gly Asp Lys Val Asn Phe Phe Arg

                20                  25

<210> 7

<211> 34

<212> DNA

<213> Artificial Sequence

<220>

<223> A positive-oriented primer designed based on the amino
      acid sequence of the C-terminus of murine GAD65

<400> 7

cagacatatg gctcaacaca caaatgtctg cttc     34

<210> 8

<211> 36

<212> DNA

<213> Artificial Sequence

<220>

<223> A reversed-oriented primer designed based on the amino

acid sequence of the C-terminus of murine GAD65

<400> 8

cggaattcgg atcctgaaac agtttgatga gtgagg     36

<210> 9

<211> 22

<212> PRT

<213> E. Coli transformed with pET14b containing the gene for

murine GAD65

<400> 9

Met Gly Ser Ser His His His His His His Ser Ser Gly Leu Val

1               5                   10                  15

Pro Arg Gly Ser His Met Val

                20